# EUROPEAN PATENT APPLICATION

(11) **EP 2 684 532 A2**
(43) Date of publication of application: **15.01.2014**
(21) Application number: 13175994.6
(22) Date of filing: 10.07.2013
(51) Int. Cl.: A61B 17/34

(54) **Surgical seal assembly including a guard member**

(30) Priority: 11.07.2012 US 201261670165 P; 25.06.2013 US 201313926348
(71) Applicant: Covidien LP, Mansfield, MA 02048 (US)
(72) Inventor: Kleyman, Gennady, Brooklyn, NY New York 11230 (US)
(74) Representative: Maschio, Antonio

(57) **Abstract**

A surgical seal assembly including a seal and a guard member cooperating with the seal, the guard member constructed as a flat element that is subsequently deformed for assembly and which, when assemble, may assume a shape of the seal so as to protect the seal from instruments inserted therethrough. By being generally flat when constructed, the guard member may be manufactured more easily and more cheaply than prior art guard components that are molded.

## Description

### CROSS REFERENCE TO RELATED APPLICATION

The present application claims the benefit of and priority to U.S. Provisional Application Serial No. 61/670,165, filed on July 11,2012, the entire contents of which are incorporated herein by reference.

### BACKGROUND

### Technical Field

The present disclosure relates to a surgical device and, more particularly, but not exclusively, relates to a surgical seal assembly for a cannula assembly and having a seal with a guard member.

### Background of Related Art

Minimally invasive surgical procedures including both endoscopic and laparoscopic procedures permit surgery to be performed on organs, tissues and vessels far removed from an opening within the tissue. Laparoscopic and endoscopic procedures generally require that any instrumentation inserted into the body be sealed, e.g., provisions must be made to ensure that gases do not enter or exit the body through the incision as, for example, in surgical procedures in which the surgical region is insufflated. These procedures typically employ surgical instruments which are introduced into the body through a cannula. The cannula has a housing at a proximal end thereof in which a seal assembly is mounted. The seal assembly provides a substantially fluid tight seal about the instrument to preserve the integrity of the established pneumoperitoneum.

Minimally invasive procedures have several advantages over traditional open surgery, including less patient trauma, reduced recovery time, reduced potential for infection, etc. However, despite its recent success and overall acceptance as a preferred surgical technique, minimally invasive surgery, such as laparoscopy, has disadvantages. In particular, the insertion of the surgical instrument within the cannula has proven to be difficult in certain procedures, e.g., in procedures requiring extensive manipulation of the long narrow endoscopic instruments within a remote site. In addition, many conventional seal assemblies are not particularly well-adapted to receive a surgical instrument if it is inserted at an angle, thus resulting in damage to the seal assemblies. This type of insertion often results in the instrument missing the target (e.g. septum seal, etc.) and becoming lodged in an undesirable location within the seal assembly. In addition, angulation and/or manipulation of instrumentation within the cannula often present difficulties with respect to maintaining seal integrity. Thus, there remains a need for an apparatus that may be used to guide a surgical instrument through a seal assembly in a more efficient and efficacious manner.

**SUMMARY**

The present invention, in accordance with various embodiments thereof, may be directed to a surgical seal assembly, comprising: a seal; and a guard member cooperating with the seal, the guard member constructed as a generally flat element that is subsequently deformed to assume a shape of the seal, the guard member adapted and dimensioned to protect at least a portion of the seal during insertion of the surgical instrument. The guard member may be flexible. The guard member may have a general frusto-conical configuration when assembled. The seal may also have a general frusto-conical configuration. The guard member may include a body portion and a lower portion, the body portion being configured as an elongated strip and the lower portion configured to include a plurality of, e.g., equally spaced apart, leaves. The plurality of equally spaced apart leaves may be separated from each other via a plurality of slits. The body portion may have a first thickness and the lower portion has a second thickness, the first thickness being greater than the second thickness. The body portion may include a proximal end and a distal end, the proximal end having a pin and the distal end having a pin-receiving opening. The guard member, upon positioning on the seal, may assume the shape of the seal such that the plurality of leaves cooperate with each other to protect the seal. Advantageously, the guard member may be manufactured by stamping.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a perspective view of a cannula assembly and a trocar assembly, with parts separated, in accordance with an example embodiment of the present invention;

FIG. 2 is a side, cross-sectional view of the seal assembly of FIG. 1, in accordance with an example embodiment of the present invention;

FIG. 3 is a top, partially cut-away view of the seal assembly of FIG. 2, in accordance with an example embodiment of the present invention;

FIG. 4 is a cross-sectional view of a portion of the seal assembly illustrating the guard member positioned on the inner seal of the seal assembly, in accordance with an example embodiment of the present invention;

FIG. 5 is a perspective view of one embodiment of the guard member in a flat configuration, in accordance with an example embodiment of the present invention;

FIG. 6 is a perspective view of the guard member of FIG. 5 in a bent configuration, in accordance with an example embodiment of the present invention;

FIG. 7 is a perspective view of the guard member of FIG. 5 in a bent configuration, where a pin engages an opening to secure the guard member in a substantially circular configuration, in accordance with an example embodiment of the present invention;

FIG. 8 is a side view of the guard member of FIG. 5, where the plurality of leaves are on the same plane with the body portion of the guard member, in accordance with an example embodiment of the present invention;

FIG. 9 is a side view of the guard member of FIG. 5, where the plurality of leaves are angled with respect to the body portion of the guard member, in accordance with an example embodiment of the present invention;

FIG. 10A is a perspective view of a guard member, where the distal ends of the guard member are semi-circular, in accordance with an example embodiment of the present invention; and

FIG. 10B is a perspective view of a guard member, where the distal ends of the guard member are rectangular, in accordance with an example embodiment of the present invention.

The figures depict preferred embodiments of the present disclosure for purposes of illustration only. One skilled in the art will readily recognize from the following discussion that alternative embodiments of the structures and methods illustrated herein may be employed without departing from the principles of the present disclosure described herein.

### DETAILED DESCRIPTION

The cannula assembly of the present disclosure, in combination with a seal system internal to the cannula assembly, provides a substantial seal between a body cavity of a patient and the outside atmosphere before, during and after insertion of a surgical instrument through the cannula assembly. Moreover, the seal assembly of the present disclosure is capable of accommodating surgical instruments of varying diameters, e.g., from 5 mm to 15 mm, by providing a fluid tight seal with each instrument when inserted. The flexibility of the present seal assembly greatly facilitates endoscopic surgery where a variety of instruments having differing diameters are often used during a single surgical procedure.

The seal assembly contemplates the introduction and manipulation of various types of instrumentation adapted for insertion through a trocar and/or cannula assembly while maintaining a fluid tight interface about the instrumentation to preserve the atmospheric integrity of a surgical procedure from gas and/or fluid leakage, e.g,, insufflation gas. Specifically, the seal assembly accommodates angular manipulation of the surgical instrument relative to the seal housing axis. This feature of the present disclosure desirably minimizes the entry and exit of gases and/or fluids to/from the body cavity. Examples of instrumentation include clip appliers, graspers, dissectors, retractors, staplers, laser probes, photographic devices, endoscopes and laparoscopes, tubes, and the like. Such instruments will be collectively referred to herein as "instruments or instrumentation."

Embodiments of the presently disclosed apparatus will now be described in detail with reference to the drawings, in which like reference numerals designate identical or corresponding elements in each of the several views. As used herein, the term "distal" refers to that portion of the tool, or component thereof which is farther from the user while the term "proximal" refers to that portion of the tool or component thereof which is closer to the user.

Reference will now be made in detail to embodiments of the present disclosure. While certain embodiments of the present disclosure will be described, it will be understood that it is not intended to limit the embodiments of the present disclosure to those described embodiments. To the contrary, reference to embodiments of the present disclosure is intended to cover alternatives, modifications, and equivalents as may be included within the spirit and scope of the embodiments of the present disclosure as defined by the appended claims.

For exemplary purposes, the access apparatus will be described in terms of a cannula assembly, which is adapted for introduction, typically utilizing a trocar, within the abdominal cavity during a laparoscopic surgical procedure. However, it is appreciated that the access apparatus may be any apparatus suitable for introduction and passage of surgical objects into underlying tissue including, e.g., catheters, trocar assemblies, endoscopic portals, hand access devices, etc., through an incision or through a natural body opening.

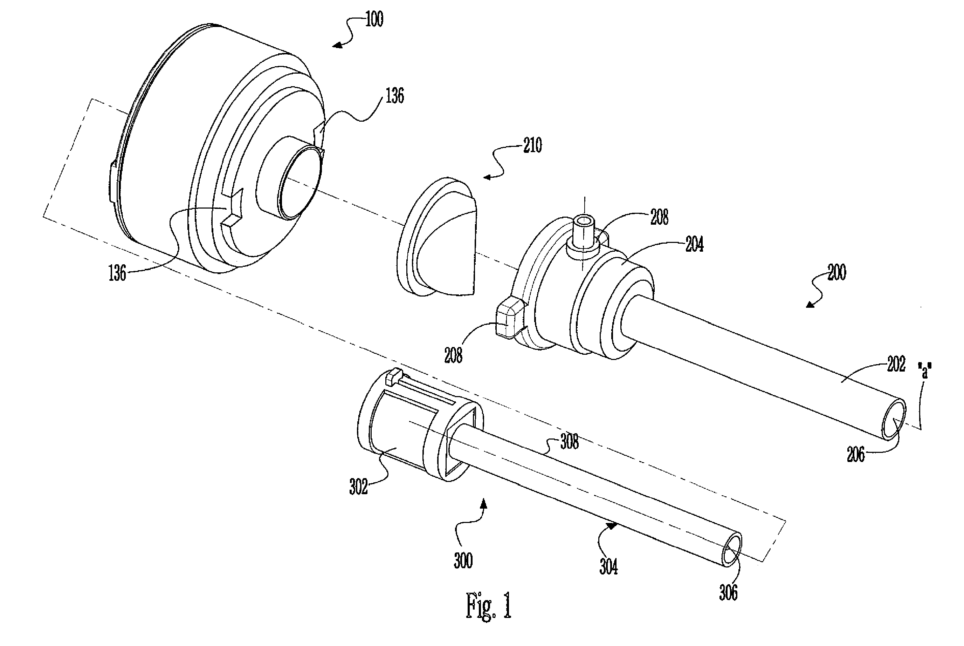

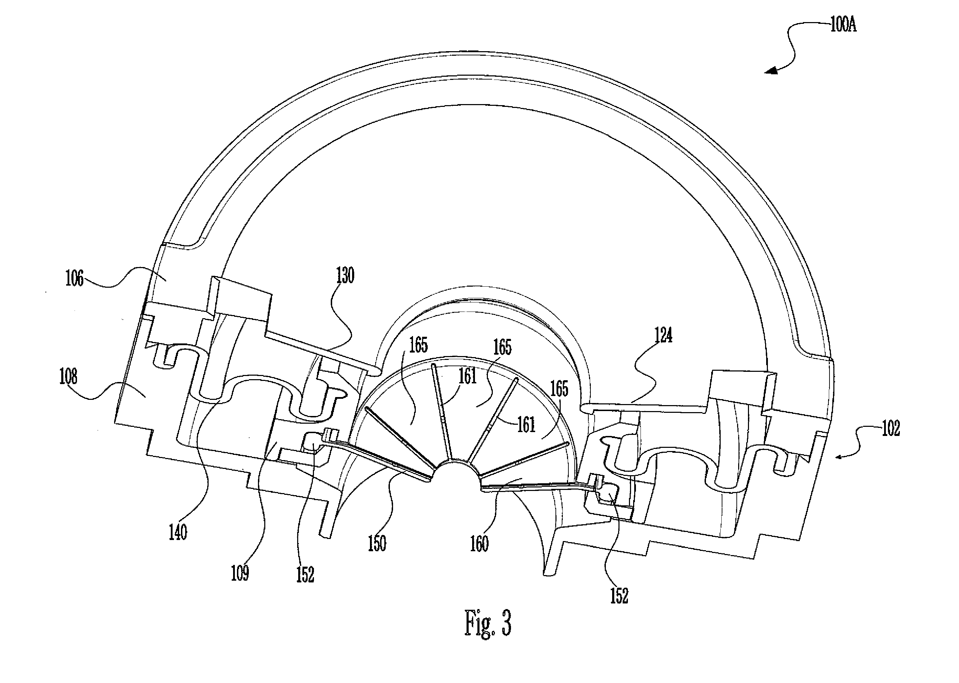

Referring now to the drawings, in which like reference numerals identify identical or substantially similar parts throughout the several views, FIGS. 1-4 illustrate a seal assembly 100.

Referring to FIG. 1, the seal assembly 100 of the present disclosure is shown with a cannula assembly 200. Cannula assembly 200 may be any conventional cannula assembly suitable for the intended purpose of accessing a body cavity and permitting introduction of instruments therethrough. Cannula assembly 200 is particularly adapted for use in laparoscopic surgery where the peritoneal cavity is insufflated with a suitable gas, e.g., CO₂, to raise the cavity wall from the internal organs therein. The cannula assembly 200 is typically used with an obturator assembly 300 which is an elongate instrument positionable within the cannula assembly 200. The obturator assembly 300 (which may be optical or nonoptical) may have a sharp end or a blunt end (a retractable sharp end is shown in FIG. 1) and is utilized to pass through, e.g., abdominal tissue, to facilitate introduction of the cannula assembly 200 within the abdominal cavity. Once access to the abdominal cavity is achieved, the obturator assembly 300 is removed from the cannula assembly 200 leaving the cannula assembly 200 in place for introduction of the surgical instrumentation utilized to perform the procedure.

Cannula assembly 200 includes cannula sleeve 202 and cannula housing 204 mounted to an end of the sleeve 202. Cannula sleeve 202 defines a longitudinal axis "a" extending along the length of sleeve 202. Sleeve 202 further defines an internal longitudinal passage 206 dimensioned to permit passage of surgical instrumentation.

Cannula housing 204 may be a multi-component element secured via a snap fit, ultrasonic welding or any other means envisioned by one skilled in the art including, e.g., adhesive means. Cannula housing 204 may further include diametrically opposed housing grips 208 dimensioned and arranged for gripping engagement by the fingers of the user. Cannula housing 204 may further include an internal duck bill or zero closure valve 210. Such zero closure valve 210 opens to permit passage of the surgical instrumentation and closes in the absence of the instrumentation. The valve may be preferably adapted to close upon exposure to the forces exerted by the insufflation gases in the internal cavity. Other zero closure valves are also contemplated including single or multiple slit valve arrangements, trumpet valves, flapper valves, etc.

Cannula sleeve 202 and cannula housing 204 may be formed of stainless steel or other rigid materials such as a polymeric material or the like. Cannula sleeve 202 and/or cannula housing 204 may be clear or opaque. The diameter of sleeve 202 may vary, but typically ranges from, e.g., 5 to 15 mm for use with the seal assembly 100 of the present disclosure.

Obturator assembly 300 includes obturator housing 302 and obturator member 304 extending from the housing 302. Obturator member 304 includes a penetrating end 306 adjacent its distal end. Penetrating end 306 may be sharp or blunt. An obturator sleeve 308 is coaxially mounted about obturator member 304. Obturator sleeve 308 may retract to expose piercing end 306. Alternatively, obturator member 304 may be advanced within obturator sleeve 308 to expose piercing end 306. Other alternate obturator mechanisms for exposing penetrating end 306 are also envisioned. Alternatively, the obturator may have other configurations, e.g., without a retractable sleeve.

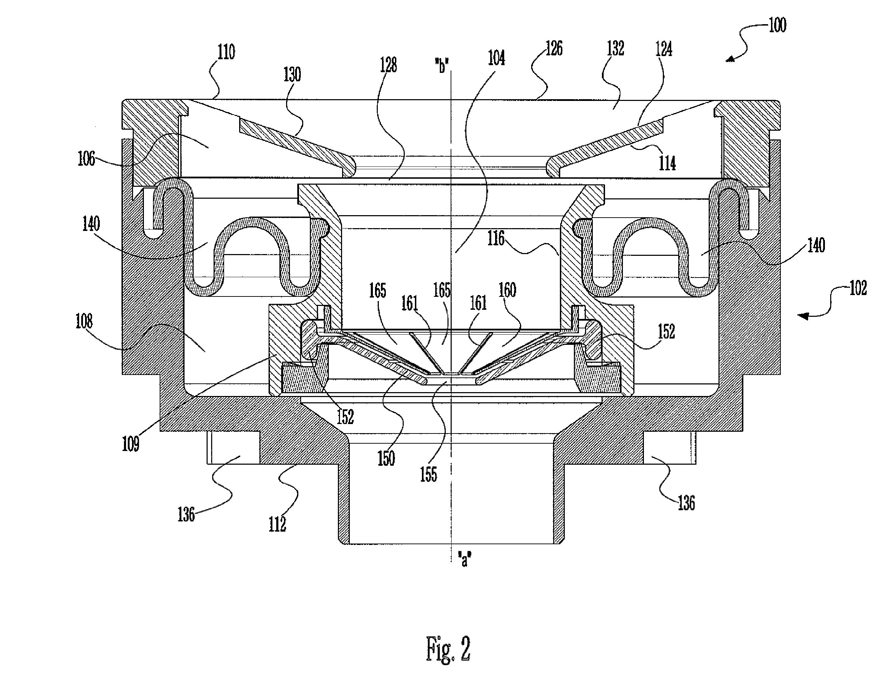

Referring now to FIGS. 2-4, top and side cross-sectional views of the seal assembly 100 of FIG. 1, illustrating the guard member 160 positioned on an inner seal 150 of the seal assembly 100, in accordance with the present disclosure is presented. It should be recognized that the inner seal 150 and the seal assembly may have any conceivable configuration, and the embodiments shown herein are merely exemplary.

Referring now to FIG. 2, in conjunction with FIG. 1, seal assembly 100 will be discussed in detail. Seal assembly 100 includes seal housing, generally identified as reference numeral 102, and longitudinal opening 104 extending through seal housing 102. Seal housing 102 houses the sealing components of the assembly and defines central seal housing axis "b" which is preferably parallel to the axis "a" of cannula sleeve 202 and, more preferably, coincident with the axis "a" of the cannula when the seal assembly 100 is mounted to the cannula assembly 200. In one embodiment, seal housing 102 may incorporate several housing components 106, 108 which, when assembled together, form the seal housing 102. Assembly of housing components 106, 108 may be effected by any suitable adhesive means including adhesives, cements or the like or mechanical means including tongue groove arrangements, bayonet couplings, interference fits, etc. Alternatively, seal housing 102 may be monolithically formed as a single unit.

Seal housing 102 defines proximal and distal ends 110, 112, respectively. Adjacent proximal end 110 is internal tapered wall 114 which extends radially inwardly toward seal housing axis "b" from proximal to distal, i.e., tapered wall 114 has both longitudinal and radial components of direction, and terminates in internal annular wall 116. Annular wall 116 serves to restrict the internal dimension of longitudinal opening 104 to at least partially constrain lateral movement of a surgical instrument (not shown) introduced through seal housing 102.

Seal housing 102 may be constructed of a plurality of different materials, including, but not limited to, polymeric, metallic, or elastomeric. Preferably, the components of seal housing 102 are formed of a polycarbonate material such as ABS available from the General Electric Company. Seal housing 102 may further include a handle which may be of any suitable ergonomic design. Moreover, seal housing 102 may be used in conjunction with, or detachably mounted, to cannula assembly 200 such as those described hereinabove.

Seal assembly 100 includes seal 150 mounted within seal housing 102. Seal 150 may be mounted within seal housing 102 through conventional means such as for example with the use of adhesives, cements or the like. Alternatively, or in conjunction with the aforementioned adhesive means, seal 150 may be disposed or trapped between housing components 106, 108 of seal housing 102 to effect the mounting.

Seal 150 may be a septum seal incorporating a circular aperture 155 formed of any suitable elastomeric material. Other arrangements for seal 150 are also envisioned. The seal 150 may be flat, conical, frusto-conical or any other suitable configuration.

Although seal 150 is disclosed as being elastomeric, it is appreciated that other seal types may be used and still achieve the objectives of the present disclosure. For example, seal 150 may be fabricated from gel, foams, or may include fluid-filled bladder seal arrangements.

Referring now to FIGS. 2-4, seal assembly 100 further includes guide 124 which provides an exterior surface of seal housing 102 preferably adjacent internal tapered wall 114. Guide 124 may be secured to the exterior surface, e.g., internal tapered wall 114 of seal housing 102. Guide 124 is generally tapered in configuration, e.g., generally frusto-conical shaped, having proximal entry opening 126 and distal exit opening 128, and inner guide portion 130 defining internal channel 132. Preferably, distal exit opening 128 defines an internal dimension greater than a corresponding internal dimension of aperture 155 of seal 150.

In use, guide 124 is adapted to generally direct the instrument toward the aperture 155 of seal 150 upon advancement of the instrument through longitudinal opening 104 of seal housing 102. In one preferred embodiment, guide 124 deflects a surgical instrument towards axis "b", such as, e.g., when an instrument is initially introduced off axis or angulated with respect to the seal housing axis "b," to substantially prevent lodging of the instrument within the guide 124. Thereafter, the tapered orientation of guide 124 guides the instrument towards the seal axis "b."

It is envisioned that seal assembly 100 may be detachably connected to cannula assembly 200. Preferably, seal housing 102 is dimensioned to be releasably mounted to cannula housing 204. In this regard, it is appreciated that seal housing 102 and cannula housing 204 may incorporate means for facilitating the releasable connection of seal assembly 100 to cannula assembly 200 including, e.g., an interference fit, bayonet coupling, screw arrangement, etc. on corresponding structure of the seal housing 102 and cannula housing 204.

For example, seal housing 102 may include locking detents 136 (see FIGS. 1 and 2) which engage corresponding structure on cannula housing 204 to secure seal assembly 100 to cannula assembly 200. Thus, the surgeon may remove seal assembly 100 from the cannula assembly 200 at any time during the surgical procedure and similarly, mount the seal assembly 100 to the cannula when desired in order to provide a sealing engagement with an instrument to be inserted through the cannula. In addition, seal assembly 100 may be readily adapted for mounting to conventional cannula of differing structures. Alternatively, seal housing 102 may be permanently secured to cannula housing 204 if desired.

Referring back to the seal 150 illustrated in FIGS. 2-4, the seal may include a connection member 152 that circumferentially extends around the top portion of the seal 150. The connection member 152 may engage a portion of housing component 109. The connection member 152 is configured to secure the seal 150 to the seal housing 102. The top, cut-away view 100A and the side, cross-section view 100B further illustrate the seal 150 having the connection member 152 securing the seal 150 to the housing component 109.

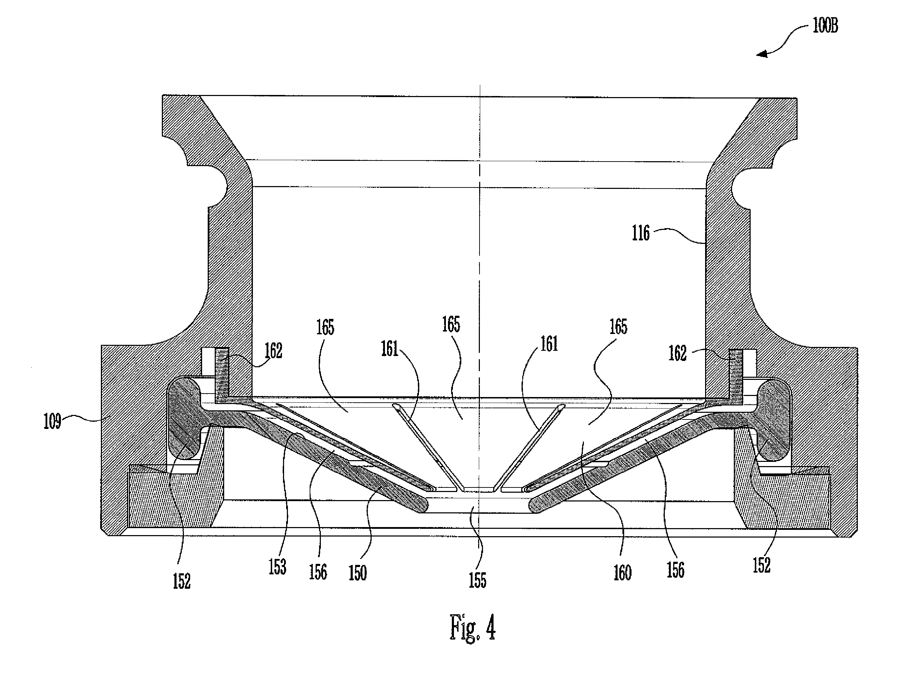

Moreover, the cross-sectional view 100B of FIG. 4 also depicts a guard member 160, a portion 162 of which is shown as engaging the seal housing component 109. The guard member portion 162 may be mounted in the vicinity of the connection member 152 of the seal 150. It is contemplated that the guard member portion 162 is in abutting relationship to the connection member 152 of the seal 150. However, one skilled in the art may contemplate a different configuration where such elements do not abut each other. Regardless, the plurality of leaves or leaves 165 are advantageously configured to assume the general shape of the seal 150, which in this exemplary case, is a frusto-conical shape. Furthermore, as shown in FIG. 4, the plurality of leaves 165 may refrain from frictionally engaging the top portion 153 of seal 150. In other words, a gap 156 may be present between the plurality of leaves 165 and the seal 150.

The housing component 109 may also be arranged to engage with or cooperate with or be associated with bellows 140. Bellows 140 may aid in centering the housing component 109, which in turn centers the seal 150 with respect to the seal housing axis "b." As illustrated bellows 140 are connected to the side wall of the seal housing 102. Of course, the bellows 140 may be optional.

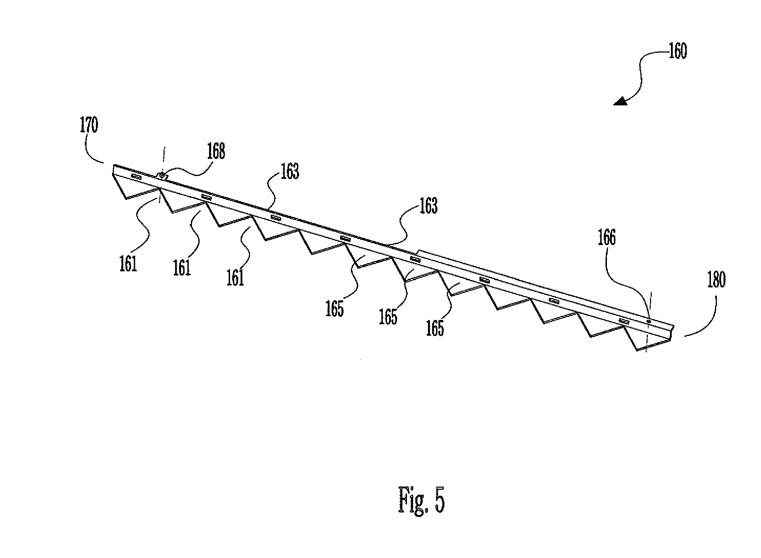

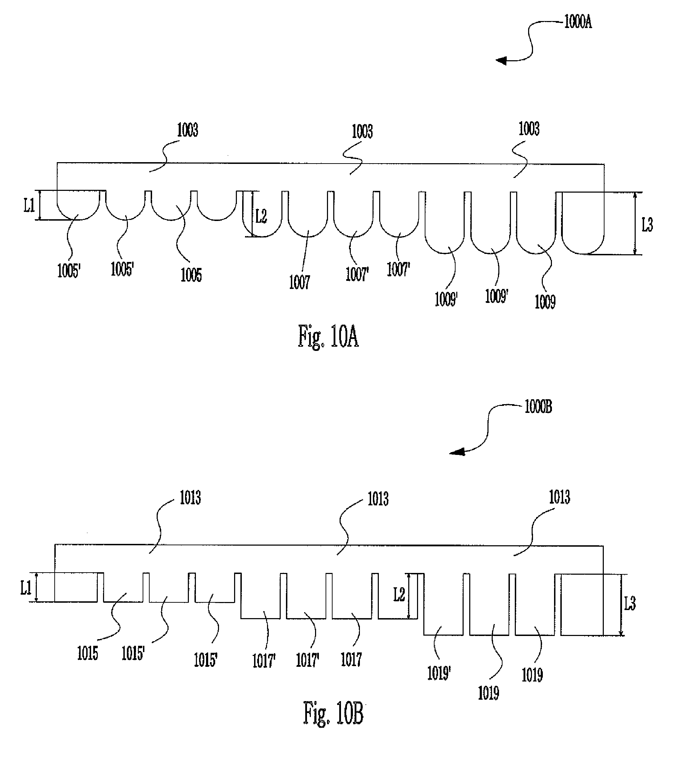

Additionally, seal 150 is adapted and dimensioned to define a general frusto-conical shape extending radially inwardly relative to the seal housing axis "b" from proximal to distal end. A guard member 160, as will be discussed in detail below with reference to FIGS. 5-9, may include a plurality of leaves 165 separated from each other by a plurality of slits 161. The plurality of adjacent leaves 165 may be in non-overlapping, overlapping or partial overlapping relation with each other. The plurality of leaves 165 are suffiently rigid so as to protect the elastomeric seal while also being suficiently flexible to deflect, bend, etc. upon engagement with the inserted surgical object. Any shape, including, but not limited to, rectangular, rounded, triangular, etc. are envisioned for the plurality of leaves 165, as will be described below with reference to FIGS. 10A-10B.

Referring to FIGS. 5-9, guard member 160 is described. In particular, referring to FIG. 5, the guard member 160 is shown in a first configuration, that is, a generally straight configuration, as a generally flat element. By being generally flat when constructed, the guard member may be manufactured more easily and more cheaply, e.g., by being stamped, than prior art guard components that are typically molded via expensive and complex injection molding equipment.

The guard member 160 includes a body portion 163 extending from a first end 170 to a second end 180 of the guard member 160. The guard member 160 also includes a plurality of leaves 165. The plurality of adjacent leaves 165 may, when configured for assembly, create a multi-leafed effect. The plurality of leaves 165 may be separated from each other by a plurality of voids or slits 161. One skilled in the art may contemplate using leaves 165 having a plurality of different shapes and of a plurality of different sizes, as discussed below with reference to FIGS. 10A-10B.

In an embodiment, the body portion 163 has a first thickness for providing improved rigidity for mounting, and the lower portion 165 has a second thickness, where the first thickness is greater than the second thickness. Additionally, the body portion 163 may include a first end 170 and a second end 180, the first end 170 having, e.g., a pin 168, and the second end 180 having, e.g., a pin receiving opening 166. The pin receiving opening 166 may be adapted and dimensioned to receive the pin 168 to provide for a secure connection therebetween.

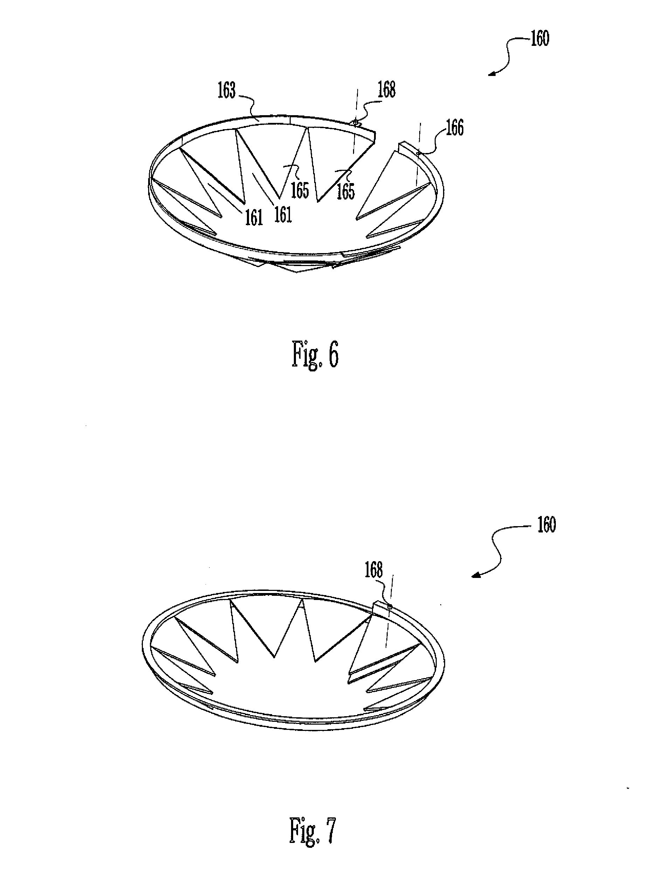

Referring now to FIG. 6, the guard member 160 is shown in a second, e.g., flexed, configuration. As stated above, the guard member 160 is a flexible member capable of being deformed into a circular configuration for assembly into, for example, the seal 150, as shown above with reference to FIGS. 1-4. Thus, the guard member 160 cooperates with the seal 150, the guard member 160 being constructed as a generally flat element that is subsequently deformed (see FIGS. 6 and 7) for assembly and that, when assembled, assumes the general shape of the seal 150. For example, the seal 150 may define a general frusto-conical configuration. As such, the guard member 160 may be deformed to assume the general frusto-conical configuration of the seal 150, as the guard member 160 is positioned or placed on the seal 150. The guard member 160, upon positioning on the seal 150, assumes the shape of the seal 150 such that the plurality of leaves 165 are arranged to protect the seal 150 and the guard member 160. As shown in FIG. 7, the pin 168 has been connected to the pin-receiving configuration 166 in order to provide a secure connection between the first and second ends 170, 180 of the guard member 160.

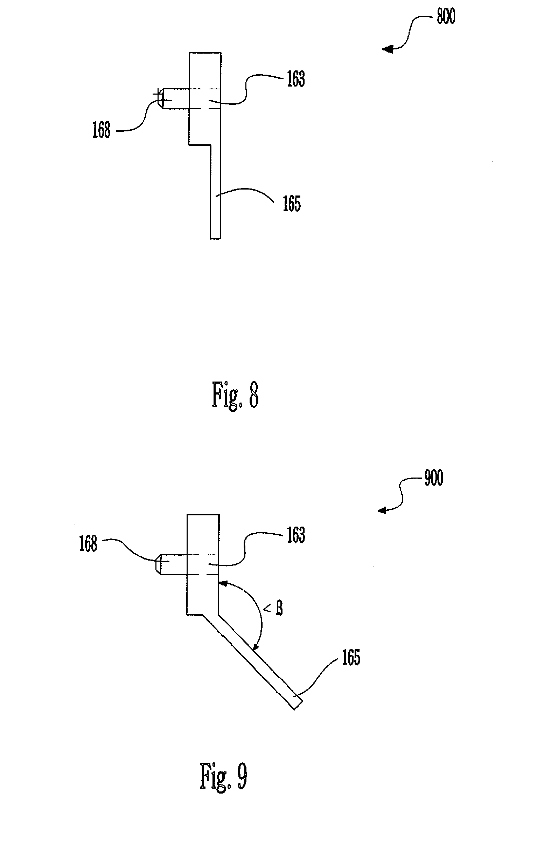

FIGS. 8-9 illustrate side views of the guard member 160. FIG. 8 illustrates the guard member 160 in a straight configuration 800, whereas FIG. 9 illustrates the guard member 160 in a bent configuration 900. In the bent configuration 900, the guard member 160 assumes a general frusto-conical configuration upon being positioned on the seal 150. Thus, the guard member 160, upon positioning on the seal 150, assumes the shape of the seal 150 such that the plurality of leaves 165 cooperate with each other to protect the seal 150.

Referring to FIGS. 10A-10B, alternative embodiments of a guard member are presented. In FIG. 10A, a guard structure 1000A is shown, where the guard structure 1000A includes an upper portion 1003 and three lower portions 1005, 1007, 1009. The lower portions 1005, 1007, 1009 include semi-circular leaves or leaves (instead of the triangular leaves of FIGS. 5-9). The first lower portion 1005 includes leaves 1005' having a first length, L₁, the second lower portion 1007 includes leaves 1007' having a second length, L₂, and the third lower portion 1009 includes leaves 1009' having a third length, L₃. in one embodiment, the third length is greater than the second length, and the second length is greater than the first length.

In FIG. 10B, a guard structure 1000B is shown, where the guard structure 1000B includes an upper portion 1013 and three lower portions 1015, 1017, 1019. The lower portions 1015, 1017, 1019 include rectangular leaves or leaves (instead of the triangular leaves of FIGS. 5-9). The first lower portion 1015 includes leaves 1015' having a first length, L₁, the second lower portion 1017 includes leaves 1017' having a second length, L₂, and the third lower portion 1019 includes leaves 1019' having a third length, L₃. In one embodiment, the third length is greater than the second length, and the second length is greater than the first length. It is contemplated that any number of different portions may be provided. Of course, one skilled in the art may contemplate a plurality of different shapes and sizes for the plurality of leaves or leaves.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of presently disclosed embodiments. Thus the scope of the embodiments should be determined by the appended claims and their legal equivalents, rather than by the examples given.

The invention may be described by reference to the following numbered paragraphs:
1. A surgical seal assembly, comprising:
   a seal; and
   a guard member cooperating with the seal, the guard member constructed as a generally flat element that is subsequently deformed to assume a shape of the seal, the guard member adapted and dimensioned to protect at least a portion of the seal during insertion of the surgical instrument.
2. The surgical seal assembly according to paragraph 1, wherein the guard member is flexible.
3. The surgical seal assembly according to paragraph 1, wherein the guard member has a general frusto-conical configuration when assembled.
4. The surgical seal assembly according to paragraph 1, wherein the seal has a general frustoconical configuration.
5. The surgical seal assembly according to paragraph 1, wherein the guard member includes a body portion and a lower portion, the body portion configured to be an elongated strip and the lower portion configured to include a plurality of equally spaced apart leaves.
6. The surgical seal assembly according to paragraph 5, wherein the plurality of equally spaced apart leaves are separated from each other via a plurality of slits.
7. The surgical seal assembly according to paragraph 5, wherein the body portion has a first thickness and the lower portion has a second thickness, the first thickness being greater than the second thickness.
8. The surgical seal assembly according to paragraph 6, wherein the body portion includes a proximal end and a distal end, the proximal end having a pin and the distal end having a pin receiving opening.
9. The surgical seal assembly according to paragraph 8, wherein the guard member, upon positioning on the seal, assumes the shape of the seal such that the plurality of leaves cooperate with each other to protect the seal.
10. The surgical seal assembly according to paragraph 1, wherein the guard member is manufactured by stamping.

## Claims

1. A surgical seal assembly, comprising:
a seal; and
a guard member cooperating with the seal, the guard member constructed as a generally flat element that is subsequently deformed to assume a shape of the seal, the guard member adapted and dimensioned to protect at least a portion of the seal during insertion of the surgical instrument.

2. The surgical seal assembly according to claim 1, wherein the guard member is flexible.

3. The surgical seal assembly according to claim 1 or claim 2, wherein the guard member has a general frusto-conical configuration when assembled.

4. The surgical seal assembly according to any of the preceding claims, wherein the seal has a general frustoconical configuration.

5. The surgical seal assembly according to any of the preceding claims, wherein the guard member includes a body portion and a lower portion, the body portion configured to be an elongated strip and the lower portion configured to include a plurality of equally spaced apart leaves.

6. The surgical seal assembly according to claim 5, wherein the plurality of equally spaced apart leaves are separated from each other via a plurality of slits.

7. The surgical seal assembly according to claim 5 or claim 6, wherein the body portion has a first thickness and the lower portion has a second thickness, the first thickness being greater than the second thickness.

8. The surgical seal assembly according to any of claims 5 to 7, wherein the body portion includes a proximal end and a distal end, the proximal end having a pin and the distal end having a pin-receiving opening.

9. The surgical seal assembly according to any of the preceding claims, wherein the guard member, upon positioning on the seal, assumes the shape of the seal such that the plurality of leaves cooperate with each other to protect the seal.

10. The surgical seal assembly according to any of the preceding claims, wherein the guard member is manufactured by stamping.
